# EUROPEAN PATENT APPLICATION

(11) **EP 4 721 802 A1**
(43) Date of publication of application: **08.04.2026**
(21) Application number: 24811402.7
(22) Date of filing: 22.05.2024
(51) Int. Cl.: A61M 25/01, A61M 25/09, A61B 17/22, A61B 17/12, A61B 34/37, A61B 17/00

(54) **VASCULAR INTERVENTION DEVICE**

(30) Priority: 25.05.2023 KR 20230067800
(71) Applicant: Perazah Inc., Ansan-si, Gyeonggi-do 15588 (KR)
(72) Inventor: SEO, Jong Tae, Ansan-si Gyeonggi-do 15588 (KR); RYU, Hwan Taek, Ansan-si Gyeonggi-do 15588 (KR); SONG, Hwa Seob, Ansan-si Gyeonggi-do 15588 (KR)
(74) Representative: Ipsilon
(86) International application number: PCT/KR2024/006923
(87) International publication number: WO 2024/242463

(57) **Abstract**

Provided is a vascular intervention device. The vascular intervention device may include a main body extending in one direction, and a plurality of guide zipper-lock units provided along a length direction of the main body and configured to guide a procedure tool inserted into a human body to the human body. Each guide zipper-lock unit may include a first zipper-lock member and a second zipper-lock member. The first zipper-lock member and the second zipper-lock member may be configured to surround an outer circumferential surface of the procedure tool in the length direction while being progressively engaged with each other during sliding-out in a length direction of the procedure tool.

## Description

### TECHNICAL FIELD

The present disclosure relates to a vascular intervention device and, more specifically, to a vascular intervention device capable of preventing in-body infection caused by contamination of a procedure tool inserted into a body during a vascular intervention procedure.

### BACKGROUND

A vascular intervention procedure is a minimally invasive procedure aimed at treating vascular diseases or cancer, and is mainly performed under X-ray fluoroscopy by percutaneously inserting a thin catheter having a diameter of several millimeters or less through a blood vessel to reach a lesion site and deliver it to a target organ for treatment. Representative treatments of vascular intervention procedures currently performed in Korea as well as worldwide include, for example, trans-arterial chemoembolization (TACE) for liver cancer, percutaneous angioplasty, and artificial vascular stent placement for aortic diseases.

Most blood vessels are branched into multiple paths or have curved shapes. Accordingly, vascular intervention procedures use overlapping insertion members having multiple stages of diameters, called a coaxial system of catheters and guide wires, to prevent damage to blood vessels. In vascular intervention procedures, master-slave type systems capable of remotely controlling a procedure tool are being used to reduce radiation exposure to an operator.

In this case, when a procedure tool such as a catheter or a guide wire is exposed to the outside as it is, it may become contaminated, and there is a concern of in-body infection caused by a contaminated procedure tool during a vascular intervention procedure.

Accordingly, there is an urgent need for a measure capable of preventing contamination of procedure tools used in vascular intervention procedures.

### DISCLOSURE

### TECHNICAL PROBLEM

A technical problem to be solved by the present disclosure is to provide a vascular intervention device capable of preventing in-body infection caused by contamination of a procedure tool inserted into a body during a vascular intervention procedure.

The technical problem to be solved by the present disclosure is not limited to the above description.

### TECHNICAL SOLUTION

In order to solve the above-mentioned technical problem, the present disclosure provides a vascular intervention device.

According to an embodiment, the vascular intervention device may include a main body extending in one direction, and a plurality of guide zipper-lock units provided along a length direction of the main body and configured to guide a procedure tool inserted into a human body. The guide zipper-lock units may each include a first zipper-lock member and a second zipper-lock member. The first zipper-lock member and the second zipper-lock member may be configured to surround an outer circumferential surface of the procedure tool in the length direction while being progressively engaged with each other during sliding-out in a length direction of the procedure tool.

According to an embodiment, the main body may include a rail frame extending in one direction, a slide block slidably coupled to the rail frame in a length direction of the rail frame, and a plurality of mounting portions provided in the length direction of the rail frame and the slide block and configured such that the guide zipper-lock units are detachably mounted thereto. The mounting portions may include fixed mounting portions respectively provided at leading ends of the rail frame and the slide block in the length direction, and a movable mounting portion provided on at least one side in the length direction of the slide block.

According to an embodiment, the procedure tool may be one of a catheter, a guidewire slid into an inner side of the catheter, a microcatheter slid into the inner side of the catheter, and a micro guidewire slid into an inner side of the microcatheter. Each pair of the catheter and the guidewire, the catheter and the microcatheter, and the microcatheter and the micro guidewire may be connected to each other in the length direction through a connector, and the connector may be mounted in the guide zipper-lock unit.

According to an embodiment, the guide zipper-lock unit further may further include: a case configured to provide an internal mounting space, the case having an opening at an upper end thereof to allow a connector to be vertically mounted thereto, the connector having, in a length direction, one side to which one procedure tool is connected and the other side to which another procedure tool is connected; a guide portion provided on one side of the case and configured to guide the one procedure tool connected to the one side in the length direction of the connector, the guide portion having an upper end opened to allow the one procedure tool to be vertically inserted thereto; and a zipper-lock fixing portion positioned on the other side of the case and configured to guide the other procedure tool connected to the other side in the length direction of the connector, the zipper-lock fixing portion having an upper side opened to allow the another procedure tool to be vertically inserted thereto, and being configured such that leading ends of the first zipper-lock member and the second zipper-lock member in the length direction are fixed thereto. The zipper-lock fixing portion may be fastened to a guide portion of another guide zipper-lock unit adjacent in the length direction while being moved in the length direction together with the first zipper-lock member and the second zipper-lock member.

According to an embodiment, the guide portions may each include fastening protrusions formed on opposite sides in a width direction. The zipper-lock fixing portion may include a first slit formed on one side in the width direction and connected to the opened upper end, and a second slit formed on the other side in the width direction and connected to an opened lower end. The fastening protrusions on the opposite sides of the guide portion adjacent in the length direction pass through the opened upper side and the opened lower side of the zipper-lock fixing portion, and one of the fastening protrusions may be inserted into the first slit and the other of the fastening protrusion may be inserted into the second slit.

According to an embodiment, the guide zipper-lock units may each further include a first pulley provided inside the case and configured such that the first zipper-lock member is wound therearound, and a second pulley provided inside the case, arranged symmetrically with the first pulley, and configured such that the second zipper-lock member is wound therearound. The first pulley and the second pulley may have coil springs respectively shaft-coupled to rotation shafts of the first pulley and the second pulley, so as to enable automatic retraction of the first zipper-lock member and the second zipper-lock member that are slid out.

According to an embodiment, the guide zipper-lock units may each further include a coupling guider. The coupling guiders may be provided between the first pulley and the second pulley and configured to guide the first zipper-lock member unwound from the first pulley and the second zipper-lock member unwound from the second pulley to meet and be engaged with each other in the length direction.

According to an embodiment, the first zipper-lock member and the second zipper-lock member, which are fixed to the zipper-lock fixing portion and positioned between the coupling guiders, may be provided, at leading end sides in the length direction, with cutouts that are vertically formed so as to allow the other procedure tool to be inserted vertically and positioned between the first zipper-lock member and the second zipper-lock member.

According to an embodiment, the first zipper-lock member may include a first body extending in one direction and made of a flexible material, a protrusion provided on one surface of the first body in a length direction, and a first groove provided at a center of the protrusion in the length direction. The second zipper-lock member may include a second body extending in one direction and made of a flexible material, a recess provided on one surface of the second body, which faces the one surface of the first body, in the length direction so as to allow the protrusion to be fitted thereto, and a second groove provided in the length direction at a center of the recess and configured to form, together with the first groove, a seating hole in which the procedure tool is seated in the length direction when the second groove meets the first groove.

According to an embodiment, the first zipper-lock member may include a first body extending in one direction and made of a flexible material, a first protrusion provided in the length direction on one side of one surface of the first body, a first socket portion provided in the length direction on the other side of the one surface of the first body, and a first groove provided in the length direction between the first protrusion and the first socket portion on the one surface of the first body. The second zipper-lock member may include: a second body extending in one direction and made of a flexible material; a second socket portion provided in the length direction on one side of one surface of the second body, the one surface of the second body facing the one surface of the first body, and the second socket portion being configured to allow the first protrusion to be fitted thereto; a second protrusion provided in the length direction on another side of the one surface of the second body to be fitted into the first socket portion; and a second groove provided in the length direction between the second socket portion and the second protrusion on the one surface of the second body. The second groove may be configured to form, together with the first groove, a seating hole in which the procedure tool is seated in the length direction when the second groove meets the first groove.

According to an embodiment, the first zipper-lock member may include a first body extending in one direction and made of a rubber magnet, and a first groove provided on one surface of the first body in the length direction. The second zipper-lock member may include a second body extending in one direction and made of a rubber magnet, and a second groove provided in the length direction on one surface of the second body, which faces the one surface of the first body. The second groove may be configured to form, together with the first groove, a seating hole in which the procedure tool is seated in the length direction when the second groove meets the first groove.

According to an embodiment, the first zipper-lock member may include a plurality of first guide blocks that extend in one direction while being joint-coupled to each other, the second zipper-lock member may include a plurality of second guide blocks that extend in one direction while being joint-coupled to each other, and the plurality of first guide blocks and the plurality of second guide blocks may have structures configured to be engaged with each other when they meet in the length direction.

### ADVANTAGEOUS EFFECTS

An embodiment of the present disclosure may include a main body extending in one direction and a plurality of guide zipper-lock units provided along a length direction of the main body and configured to guide a procedure tool inserted into a human body to the inside of the human body. The guide zipper-lock units may each include a first zipper-lock member and a second zipper-lock member. The first zipper-lock member and the second zipper-lock member may be configured to surround an outer circumferential surface of the procedure tool in the length direction while being progressively be engaged with each other during sliding-out in the length direction of the procedure tool.

Accordingly, it may be possible to provide a vascular intervention device capable of preventing an intra-body infection caused by contamination of a procedure tool inserted into the human body during a vascular intervention procedure.

In addition, according to an embodiment of the present disclosure, an environment in which an operator is exposed to radiation may be minimized through remote control.

That is, according to an embodiment of the present disclosure, the safety of the vascular intervention procedure may be improved.

### DESCRIPTION OF DRAWINGS

FIG. 1 is a view illustrating a vascular intervention procedure system in which a vascular intervention device according to a first embodiment of the present disclosure is used.
FIG. 2 is a view illustrating the vascular intervention device according to the first embodiment of the present disclosure.
FIG. 3 is a view illustrating a main body of the vascular intervention device according to the first embodiment of the present disclosure.
FIGS. 4 to 6 are views sequentially illustrating a process in which a procedure tool is mounted to a guide zipper-lock unit of the vascular intervention device according to the first embodiment of the present disclosure.
FIGS. 7 to 11 are views illustrating a first zipper-lock member and a second zipper-lock member of the vascular intervention device according to the first embodiment of the present disclosure.
FIGS. 12 to 17 are views illustrating the guide zipper-lock unit of the vascular intervention device according to the first embodiment of the present disclosure.
FIGS. 18 to 22 are views illustrating a first zipper-lock member and a second zipper-lock member of the vascular intervention device according to a second embodiment of the present disclosure.
FIGS. 23 to 25 are views illustrating a first zipper-lock member and a second zipper-lock member of the vascular intervention device according to a third embodiment of the present disclosure.
FIGS. 26 to 28 are views illustrating a first zipper-lock member and a second zipper-lock member of the vascular intervention device according to a fourth embodiment of the present disclosure.

### MODE FOR INVENTION

Hereinafter, embodiments of the present disclosure will be described in detail with reference to the accompanying drawings. However, the technical idea of the present disclosure is not limited to the embodiments described herein, and may be implemented in other forms. Rather, the embodiments disclosed herein are provided so as to make this disclosure thorough and complete and to help a person ordinarily skilled in the art fully understand the concept of the present disclosure.

In this specification, when a component is described as being on another component, it means that the component may be directly formed on the other component or a third component may be interposed therebetween. In addition, in the drawings, shapes and sizes are exaggerated for effective explanation of the technical contents.

Furthermore, although terms such as first, second, and third are used in various embodiments of this specification to describe various components, the components should not be limited by such terms. These terms are merely used to distinguish one component from other components. Accordingly, a component referred to as a first component in one embodiment may be referred to as a second component in another embodiment. Each embodiment described and exemplified herein also includes an complementary embodiment thereof. In this specification, the term "and/or" is used to mean at least one of the components listed before and after the term.

In this specification, a singular expression includes a plural expression unless the context clearly indicates otherwise. In addition, terms such as "include" or "have" are intended to specify that features, numbers, steps, components, or combinations thereof described in the specification are present, and are not to be construed as excluding the possibility of the existence or addition of one or more other features, numbers, steps, components, or combinations thereof. In this specification, the term "connection" is used in a sense of including both indirect connection and direct connection between a plurality of components.

In addition, terms such as "unit," "device," and "module" described in the specification refer to a unit that processes at least one function or operation, and may be implemented by hardware, software, or a combination of hardware and software.

In addition, in the following description of the present disclosure, detailed descriptions of related well-known functions or configurations will be omitted when it is determined that such descriptions may unnecessarily obscure the gist of the present disclosure.

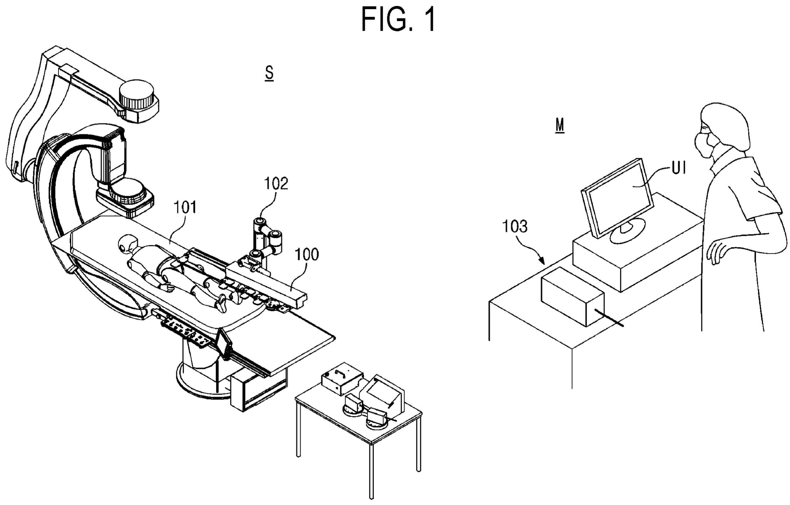

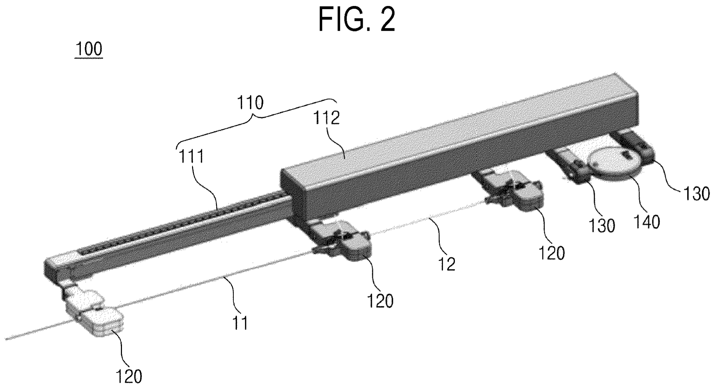

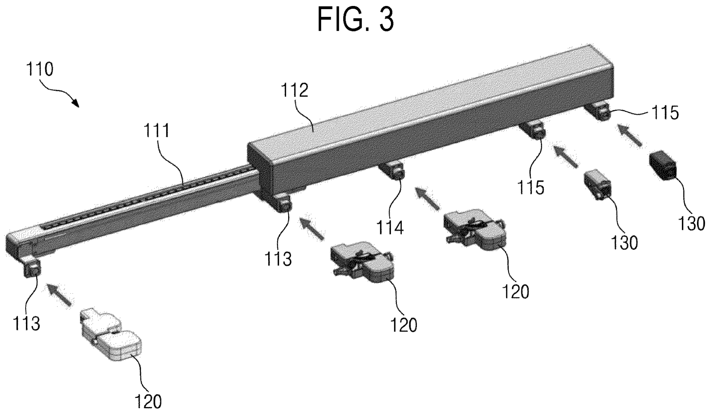

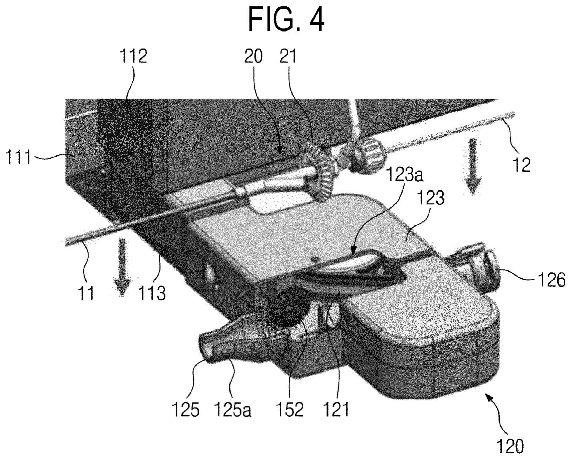

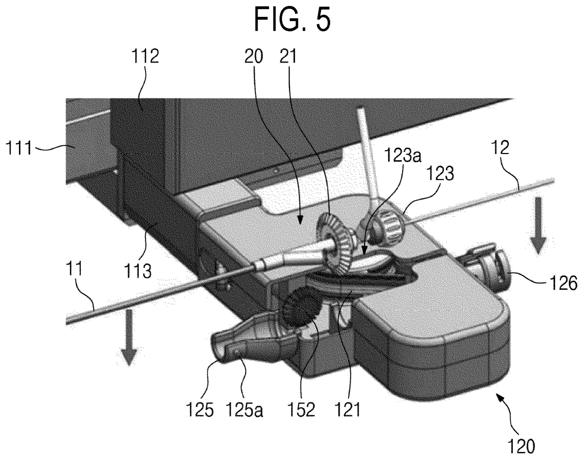

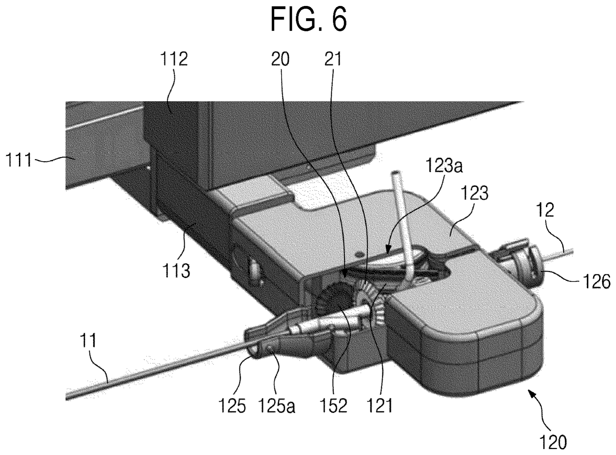

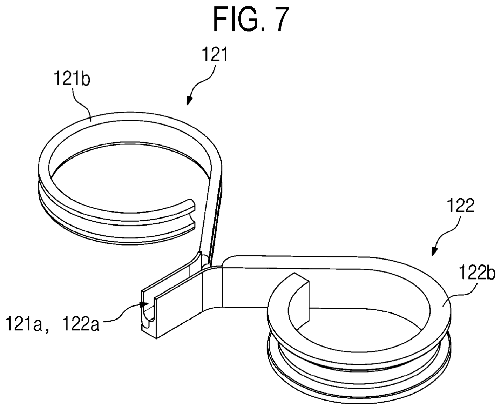

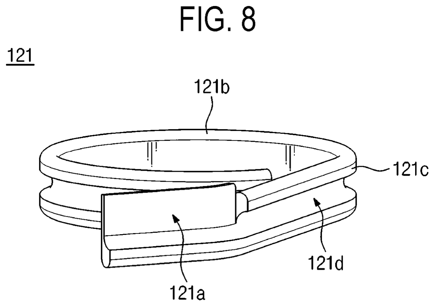

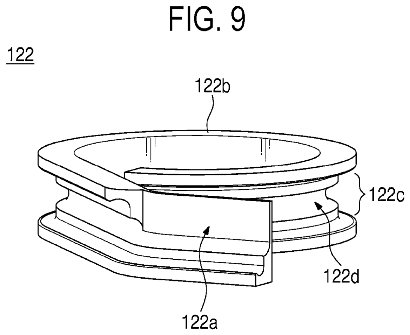

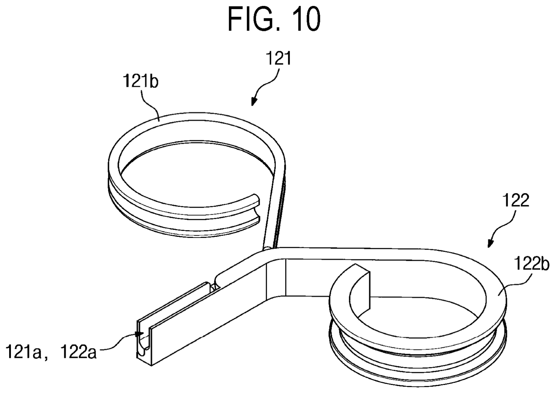

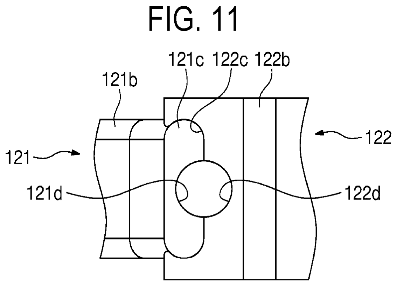

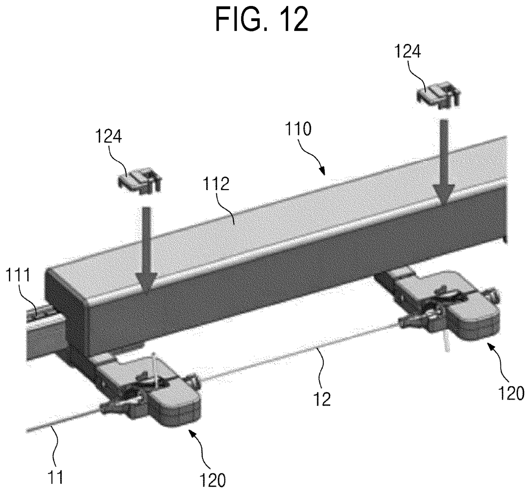

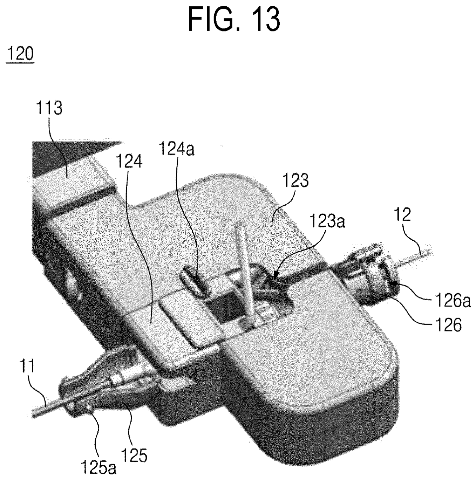

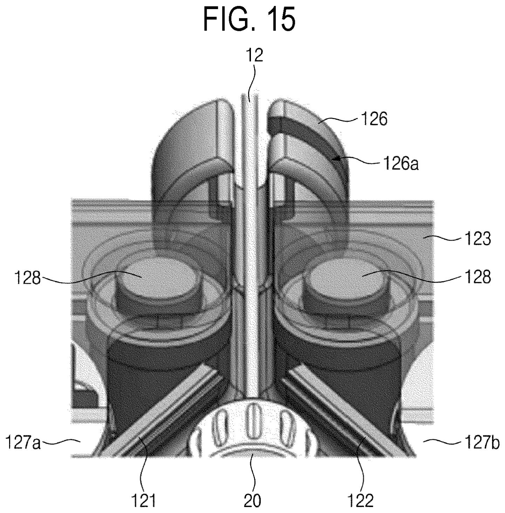

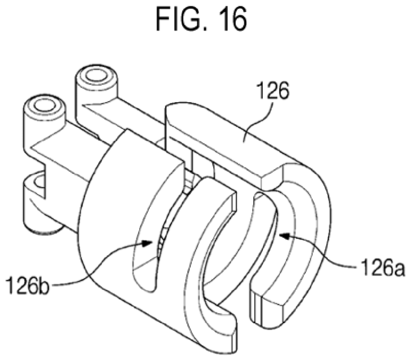

FIG. 1 is a view illustrating a vascular intervention procedure system in which a vascular intervention device according to a first embodiment of the present disclosure is used, FIG. 2 is a view illustrating the vascular intervention device according to the first embodiment of the present disclosure, and FIG. 3 is a view illustrating a main body of the vascular intervention device according to the first embodiment of the present disclosure. FIGS. 4 to 6 are views sequentially illustrating a process in which a procedure tool is mounted to a guide zipper-lock unit of the vascular intervention device according to the first embodiment of the present disclosure, FIGS. 7 to 11 are views illustrating a first zipper-lock member and a second zipper-lock member of the vascular intervention device according to the first embodiment of the present disclosure, and FIGS. 12 to 17 are views illustrating the guide zipper-lock unit of the vascular intervention device according to the first embodiment of the present disclosure.

As illustrated in FIG. 1, a vascular intervention device 100 according to a first embodiment of the present disclosure may be applied to a vascular intervention procedure system configured as a remote procedure system based on a master-slave device.

In the vascular intervention procedure system, an operator remotely controls a procedure through a master device (M), and a slave device (S) performs the procedure on a patient according to the operator's remote control. Accordingly, an environment in which the operator is exposed to radiation may be minimized.

The vascular intervention device 100 according to the first embodiment of the present disclosure may be included in the slave device (S) and may be remotely controlled by the master device (M). At this time, the slave device (S) may further include a bed 101 and a frame 102.

The bed 101 may provide a procedure surface on which a patient may lie down to receive the procedure in a supine position. The frame 102 may be installed on the bed 101 so as to be movable.

The vascular intervention device 100 according to the first embodiment of the present disclosure may be mounted on the frame 102 installed on the bed 101. In this case, the vascular intervention device 100 according to the first embodiment of the present disclosure may be mounted such that the vascular intervention device 100 is capable of rotating or translating with respect to the frame 102.

The vascular intervention device 100 according to the first embodiment of the present disclosure may rotate a procedure tool in a roll direction or may translate the procedure tool forward or backward in order to insert the procedure tool into a body and move the procedure tool to a target vessel. In addition, the vascular intervention device 100 according to the first embodiment of the present disclosure may simultaneously translate the procedure tool while rotating the procedure tool.

An operator may insert a procedure tool, which is operated by the vascular intervention device 100 according to the first embodiment of the present disclosure, into a target vessel while remotely controlling the vascular intervention device 100 according to the first embodiment of the present disclosure through the master device (M).

Here, the procedure tool may be one of a catheter inserted into a human body, a guidewire slid into the catheter, a microcatheter slid into an inner side of the catheter, and a micro guidewire slid into the inner side of the microcatheter.

Meanwhile, the master device (M) may include a display unit configured to output a user interface (UI) related to the procedure tool and a procedure process, and a manipulator 103 configured to generate a remote-control signal for operating the procedure tool according to an operator's manipulation.

The master device (M) may provide the remote-control signal generated by the operator's manipulation of the manipulator 103 to the vascular intervention device 100 according to the first embodiment of the present disclosure included in the slave device (S), and may remotely operate the procedure tool driven thereby. As such, by remotely controlling the vascular intervention device 100, radiation exposure to the operator may be minimized.

As illustrated in FIG. 2, the vascular intervention device 100 according to the first embodiment of the present disclosure, which is applied to such a vascular intervention procedure system, may include a main body 110 and a guide zipper-lock unit 120.

The main body 110 may extend in one direction. Accordingly, the main body 110 may allow a plurality of guide zipper-lock units 120 to be aligned in a length direction.

According to the first embodiment of the present disclosure, the main body 110 may include a rail frame 111, a slide block 112, and a mounting portion.

The rail frame 111 may be provided in a bar shape extending in one direction.

The slide block 112 may be coupled to the rail frame 111. In this case, the slide block 112 may be coupled to the rail frame 111 to be slidable in the length direction of the rail frame 111.

When the slide block 112 slides forward along the rail frame 111, the guide zipper-lock units 120 connected to the slide block 112 may be moved forward. Accordingly, a procedure tool mounted on the guide zipper-lock units 120 may move toward a target vessel while translating forward.

In addition, when the slide block 112 slides rearward along the rail frame 111, the guide zipper-lock units 120 connected to the slide block 112 may be moved rearward. Accordingly, the procedure tool mounted to the guide zipper-lock units 120 may be slid out of the human body while translating rearward.

Meanwhile, although not illustrated, the slide block 112 may be provided therein with a drive device configured to slide the slide block 112 with respect to the rail frame 111, a drive device configured to translate the guide zipper-lock unit 120 connected to the slide block 112 forward or rearward, and a drive device configured to roll-rotate a procedure tool mounted on the guide zipper-lock units 120.

A plurality of mounting portions may be provided in the length direction of the rail frame 111 and the slide block 112. The guide zipper-lock units 120 may be detachably mounted to the mounting portions.

As illustrated in FIG. 3, according to the first embodiment of the present disclosure, the mounting portions may be classified into a fixed mounting portion 113 and a movable mounting portion 114.

The fixed mounting portion 113 may be provided at a leading end in the length direction of each of the rail frame 111 and the slide block 112.

According to the first embodiment of the present disclosure, a first procedure tool 11 may be supported in the length direction by a guide zipper-lock unit 120 mounted on the fixed mounting portion 113 provided at the leading end of the rail frame 111 in the length direction, and a guide zipper-lock unit 120 mounted on the fixed mounting portion 113 provided at the leading end of the slide block 112 in the length direction. The first procedure tool 11 may be, for example, a catheter.

The movable mounting portion 114 may be provided on at least one side in the length direction of the slide block 112. The movable mounting portion 114 may be provided so as to be movable in the length direction of the slide block 112.

According to the first embodiment of the present disclosure, a second procedure tool 12 may be supported in the length direction by the guide zipper-lock unit 120 mounted on the fixed mounting portion 113 provided at the leading end of the slide block 112 in the length direction, and the guide zipper-lock unit 120 mounted on the movable mounting portion 114 provided on one side in the length direction of the slide block 112. The second procedure tool 12 may be, for example, a guidewire.

Accordingly, in a state in which the slide block 112 slides forward along the rail frame 111 and the first procedure tool 11 is translated forward, when the movable mounting portion 114 provided on one side in the length direction of the slide block 112 is moved forward such that the guide zipper-lock unit 120 mounted thereto is operated in conjunction therewith, the second procedure tool 12 may translate forward. That is, the guidewire may be slid into an inner side of the catheter.

Meanwhile, according to the first embodiment of the present disclosure, the main body 110 may further include a fixing portion 115.

The fixing portion 115 may be provided at a rear end side in the length direction of the slide block 112. According to the first embodiment of the present disclosure, a pair of fixing portions 115 may be provided.

A power transmission unit 130 configured to transmit power to a bobbin unit 140 may be fixed to each of the pair of fixing portions 115. Here, a third procedure tool (not illustrated) mounted on the guide zipper-lock unit 120 mounted on the movable mounting portion 114 provided on one side in the length direction of the slide block 112 may be wound around the bobbin unit 140.

Accordingly, the bobbin unit 140 may wind or unwind the third procedure tool while being rotated clockwise or counterclockwise by the power transmission unit 130. Through this, the third procedure tool (not illustrated) may translate forward or rearward. Here, the third procedure tool (not illustrated) may be, for example, a microcatheter slid into an inner side of the catheter provided as the first procedure tool 11.

A plurality of guide zipper-lock units 120 may be provided in the length direction of the main body 110.

Among the plurality of guide zipper-lock units 120, one may be mounted on the fixed mounting portion 113 provided at the leading end of the rail frame 111 in the length direction.

In addition, among the plurality of guide zipper-lock units 120, another one may be mounted on the fixed mounting portion 113 provided at the leading end of the slide block 112 in the length direction.

Further, among the plurality of guide zipper-lock units 120, still another one may be mounted on the movable mounting portion 114 provided on one side in the length direction of the slide block 112.

In this manner, the plurality of guide zipper-lock units 120 may be mounted on the fixed mounting portions 113 and the movable mounting portion 114, which are provided in the length direction of the rail frame 111 and the slide block 112, respectively, and may thereby be aligned in the length direction of the main body 110.

According to the first embodiment of the present disclosure, the guide zipper-lock units 120 may guide a procedure tool inserted into a human body to the inside of the human body.

In this case, the procedure tool may be vertically mounted to the guide zipper-lock units 120. In addition, the procedure tool mounted on the guide zipper-lock units 120 may be easily separated from the guide zipper-lock unit 120 while being lifted upward.

As illustrated in FIGS. 4 to 6, according to the first embodiment of the present disclosure, the first procedure tool 11 and the second procedure tool 12 may be vertically mounted on the guide zipper-lock units 120 in a state in which the first procedure tool 11 and the second procedure tool 12 are connected in the length direction through a connector 20.

For example, a catheter prepared as the first procedure tool 11 and a guidewire prepared as the second procedure tool 12 may be connected in the length direction through the connector 20 and then may be vertically mounted on the guide zipper-lock units 120.

In this case, a connector 20 in which the first procedure tool 11 is substantially connected to one side in the length direction and the second procedure tool 12 is connected to the other side in the length direction may be mounted on the guide zipper-lock units 120.

The first procedure tool 11, whose rear end in the length direction is connected to the connector 20, may be guided by the guide zipper-lock units 120 in a form in which the first procedure tool 11 is slid out forward from the guide zipper-lock units 120.

In addition, the second procedure tool 12, whose front end in the length direction is connected to the connector 20, may be guided by the guide zipper-lock units 120 in a form in which the second procedure tool 12 is slid out rearward from the guide zipper-lock units 120.

As illustrated in FIGS. 7 to 11, according to the first embodiment of the present disclosure, a guide zipper-lock unit 120 may include a first zipper-lock member 121 and a second zipper-lock member 122.

For example, the front end of the second procedure tool 12 in the length direction may be connected to a connector 20 that is vertically mounted on the guide zipper-lock unit 120 mounted on the fixed mounting portion 113 provided at the leading end in the length direction of the slide block 112, and the rear end of the second procedure tool 12 in the length direction may be connected to a connector 20 that is vertically mounted on the guide zipper-lock unit 120 mounted on the movable mounting portion 114 provided on one side in the length direction of the slide block 112.

Accordingly, the second procedure tool 12 may be exposed to the outside in the length direction, and therefore, the second procedure tool 12 may become contaminated.

According to the first embodiment of the present disclosure, the first zipper-lock member 121 and the second zipper-lock member 122 may be provided to surround an outer circumferential surface of the procedure tool in the length direction while being progressively engaged with each other, that is, being engaged in a so-called zipper-lock manner, during sliding-out in the length direction of the procedure tool.

Accordingly, the procedure tool may be prevented from being exposed to the outside, thereby preventing in-body infection caused by contamination of the procedure tool.

According to the first embodiment of the present disclosure, the first zipper-lock member 121 may include a cutout 121a formed in a vertical direction at a leading end side in the length direction so that the first zipper-lock member 121 may be positioned between the procedure tool and the second zipper-lock member 122 while the procedure tool is inserted in the vertical direction. The cutout 121a may form a "U"-shaped groove that allows insertion of the procedure tool in the vertical direction when the cutout 121a meets the cutout 122a provided at a leading end side of the second zipper-lock member 122 in the length direction.

According to the first embodiment of the present disclosure, the first zipper-lock member 121 may include a first body 121b, a protrusion 121c, and a first groove 121d.

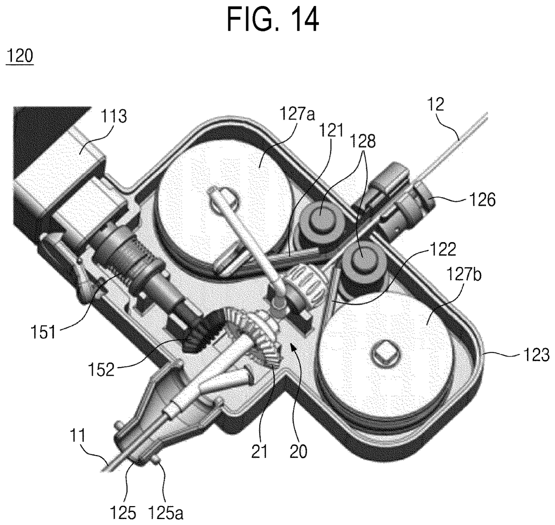

The first body 121b forms an external appearance of the first zipper-lock member 121. The first body 121b may extend in one direction. In this case, the first body 121b may extend to a length that allows the procedure tool to be covered in the length direction. The first body 121b may be formed of a flexible material so as to be wound around a first pulley (127a of FIG. 14), which will be described later.

The protrusion 121c may be provided in the length direction on one surface of the first body 121b. Here, the one surface of the first body 121b may be a surface that comes into close contact with and engages with one surface of the second zipper-lock member 122.

The protrusion 121c may be fitted into a recess 122c of the second zipper-lock member 122.

The first groove 121d may be provided in the length direction at a center of the protrusion 121c. The first groove 121d may form a seating hole in which the procedure tool is seated in the length direction when the first groove 121d meets a second groove 122d of the second zipper-lock member 122.

According to the first embodiment of the present disclosure, the second zipper-lock member 122 may be paired with the first zipper-lock member 121 to surround the procedure tool in the length direction, and thereby prevent the procedure tool from being exposed to the outside.

A cutout 122a may be provided at a leading end side of the second zipper-lock member 122 in the length direction so as to form a "U"-shaped groove that allows insertion of the procedure tool in the vertical direction when the cutout 122a meets the cutout 121a provided at the leading end side of the first zipper-lock member 121 in the length direction.

The second zipper-lock member 122 may include a second body 122b, the recess 122c, and the second groove 122d.

The second body 122b forms an external appearance of the second zipper-lock member 122. The second body 122b may extend in one direction. In this case, the second body 122b may extend in a length that allows the procedure tool to be covered in the length direction. For example, the second body 122b may be provided to have the same length as the first body 121b of the first zipper-lock member 121.

The second body 122b may be formed of a flexible material to be wound around a second pulley (127b of FIG. 14), which will be described later.

The recess 122c may be provided in the length direction on one surface of the second body 122b facing one surface of the first body 121b so that the protrusion 121c of the first zipper-lock member 121 may be fitted thereinto.

The second groove 122d may be provided in the length direction at a center of the recess 122c. The second groove 122d may form a seating hole in which the procedure tool is seated in the length direction when the second groove 122d meets the first groove 121d of the first zipper-lock member 121.

According to the first embodiment of the present disclosure, through a male-female engagement between the protrusion 121c of the first zipper-lock member 121 and the recess 122c of the second zipper-lock member 122, the first zipper-lock member 121 and the second zipper-lock member 122 may surround an outer circumferential surface of the procedure tool in the length direction while being progressively engaged with each other during sliding-out in the length direction of the procedure tool.

Meanwhile, as illustrated in FIGS. 12 to 17, according to the first embodiment of the present disclosure, the guide zipper-lock unit 120 may further include a case 123, a guide portion 125, and a zipper-lock fixing portion 126.

The case 123 forms an external appearance of the guide zipper-lock unit 120. A mounting space may be provided inside the case 123 in which a connector 20 is mounted such that one procedure tool, for example, the first procedure tool 11, is connected to one side in the length direction and another procedure tool, the second procedure tool 12, is connected to the other side in the length direction.

In this case, the case 123 may be provided, in an upper end, with an opening 123a so as to allow the connector 20 to be vertically mounted in the case 123.

According to the first embodiment of the present disclosure, the guide zipper-lock unit 120 may further include an upper cover 124. The upper cover 124 may be mounted on the case 123 so as to cover the opening 123a after the connector 20 is vertically mounted in the case 123 through the opening 123a, thereby preventing the connector 20 from being separated upward.

In this case, the upper cover 124 may be provided with a locking lever 124a that is coupled to the case 123 so as to prevent the upper cover 124 installed on the case 123 from being separated from the case 123. The locking lever 124a may be coupled to and decoupled from the case 123, and through this, the upper cover 124 may be freely installed on or removed from the case 123.

According to the first embodiment of the present disclosure, when the guide zipper-lock unit 120 is mounted on a mounting portion, for example, a fixed mounting portion 113, a driving motor 151 of a driving device provided inside the main body 110 and a driving gear 152 that is shaft-coupled to a rotary shaft of the driving motor 151 may be inserted into an internal space of the case 123.

In this case, when the connector 20 is mounted in the case 123, a gear 21 provided on the connector 20 may form a single bevel gear while being connected to the driving gear 152. At this time, the gear 21 provided on the connector 20 may be shaft-coupled to an insertion tube of the connector 20 into which a rear end of the first procedure tool 11 in the length direction is inserted.

Accordingly, when the driving motor 151 operates, rotational force provided from the driving motor 151 may be transmitted to the gear 21 through the driving gear 152, and through this, as the gear 21 rotates, the first procedure tool 11 may be interlocked therewith and may rotate about the length direction as an axis, that is, may be roll-rotated.

The guide portion 125 may be provided on one side of the case 123. According to the first embodiment of the present disclosure, the guide portion 125 may be provided on a front side of the case 123.

The guide portion 125 may guide one procedure tool, for example, the first procedure tool 11, which is connected to one side in the length direction of the connector 20 mounted on the case 123.

In this case, when the connector 20 is vertically mounted on the case 123, the guide portion 125 vertically faces the first procedure tool 11 connected to one side in the length direction of the connector 20.

Accordingly, the guide portion 125 may have an upper end opened so that the first procedure tool 11, whose rear end in the length direction is connected to one side in the length direction of the connector 20, may be vertically inserted.

As such, the opened upper end of the guide portion 125 may be connected to the opening 123a provided in the upper end of the case 123.

According to the first embodiment of the present disclosure, the guide portion 125 may include a fastening protrusion 125a.

The fastening protrusion 125a may be formed on opposite sides of the guide portion 125 in a width direction. A zipper-lock fixing portion 126 of an adjacent guide zipper-lock unit 120 is fastened to the guide portion 125 through the fastening protrusions 125a, and this will be described in more detail below.

The zipper-lock fixing portion 126 may be provided on the other side of the case 123. According to the first embodiment of the present disclosure, the zipper-lock fixing portion 126 may be provided on a rear side of the case 123. Through this, the zipper-lock fixing portion 126 may be aligned in one direction with the guide portion 125. In this case, unlike the guide portion 125 that is integrally formed with the case 123, the zipper-lock fixing portion 126 may not be coupled to the case 123, and may thus enable independent movement.

The zipper-lock fixing portion 126 may guide another procedure tool, for example, the second procedure tool 12, which is connected to the other side in the length direction of the connector 20 mounted on the case 123.

In this case, when the connector 20 is vertically mounted on the case 123, the zipper-lock fixing portion 126 may vertically face the second procedure tool 12 connected to the other side in the length direction of the connector 20.

Accordingly, the zipper-lock fixing portion 126 may have an upper end opened so that the second procedure tool 12, whose leading end in the length direction is connected to the other side in the length direction of the connector 20, may be vertically inserted.

As such, the opened upper end of the zipper-lock fixing portion 126 may be connected to the opening 123a provided in the upper portion of the case 123.

According to the first embodiment of the present disclosure, the leading ends of the first zipper-lock member 121 and the second zipper-lock member 122 in the length direction, in which the cutouts 121a and 122a are formed, may be fixed to the zipper-lock fixing portion 126.

Accordingly, the zipper-lock fixing portion 126 may be fastened to the guide portion 125 of another guide zipper-lock unit 120 adjacent in the length direction, while moving in the length direction together with the first zipper-lock member 121 and the second zipper-lock member 122.

From another perspective, the zipper-lock fixing portion 126 may be fastened to the guide portion 125 of another guide zipper-lock unit 120 adjacent in the length direction while being moved in the length direction so as to pull the first zipper-lock member 121 and the second zipper-lock member 122.

Accordingly, the procedure tool may be prevented from being exposed to the outside, and through this, in-body infection caused by contamination of the procedure tool during a vascular intervention procedure may be prevented.

Here, the movement of the zipper-lock fixing portion 126 may be performed by an operator.

As such, the zipper-lock fixing portion 126 may include a first slit 126a and a second slit 126b configured to be fastened to the guide portion 125 of another adjacent guide zipper-lock unit 120.

The first slit 126a may be formed on one side of the zipper-lock fixing portion 126 in the width direction. The first slit 126a may be connected to the opened upper end of the zipper-lock fixing portion 126.

In addition, the second slit 126b may be formed on the other side of the zipper-lock fixing portion 126 in the width direction. The second slit 126b may be connected to the opened lower end of the zipper-lock fixing portion 126.

According to the first embodiment of the present disclosure, the zipper-lock fixing portion 126 moved from one guide zipper-lock unit 120 may be fastened to the guide portion 125 provided in another guide zipper-lock unit 120 adjacent in the length direction through the first slit 126a and the second slit 126b, which are engaged with the fastening protrusions 125a of the guide portion 125 provided in the other guide zipper-lock unit 120 adjacent in the length direction.

With reference to the guide portion 125, the fastening protrusions 125a on opposite sides of the guide portion 125 may pass through the opened upper end and lower end of the zipper-lock fixing portion 126 provided in another guide zipper-lock unit 120 adjacent in the length direction, and then one fastening protrusion 125a may be inserted into the first slit 126a of the zipper-lock fixing portion 126, and the other fastening protrusion 125a may be inserted into the second slit 126b of the zipper-lock fixing portion 126. Through this, the guide portion 125 and the zipper-lock fixing portion 126 of guide zipper-lock units 120 adjacent in the length direction may be fastened to each other.

An operator may move the zipper-lock fixing portion 126 provided in one guide zipper-lock unit 120 toward another guide zipper-lock unit 120 adjacent in the length direction by holding the zipper-lock fixing portion 126 so that an outer circumferential surface of a procedure tool is surrounded by the first zipper-lock member 121 and the second zipper-lock member 122.

At this time, when the zipper-lock fixing portion 126 is positioned at a leading end side of the guide portion 125 provided on a front side of another guide zipper-lock unit 120 adjacent in the length direction, an operator may, for example, rotate the zipper-lock fixing portion 126 clockwise. Accordingly, an opened upper end and an opened lower end of the zipper-lock fixing portion 126 may be placed on the same line as fastening protrusions 125a formed on opposite sides of the guide portion 125 in a width direction.

Accordingly, the zipper-lock fixing portion 126 may be fitted on the outside of the guide portion 125 through one fastening protrusion 125a that is slidingly fastened to the opened upper end and the other fastening protrusion 125a that is slidingly fastened to the opened lower end.

In this state, the operator may rotate the zipper-lock fixing portion 126 counterclockwise. Accordingly, one fastening protrusion 125a of the guide portion 125 may be inserted into the first slit 126a of the zipper-lock fixing portion 126, and the other fastening protrusion 125a of the guide portion 125 may be inserted into the second slit 126b of the zipper-lock fixing portion 126.

Through this, movement of the zipper-lock fixing portion 126 in the length direction may be restricted. That is, escape of the zipper-lock fixing portion 126 from the guide portion 125 of the adjacent guide zipper-lock unit 120 may be prevented.

Accordingly, during a vascular intervention procedure, the first zipper-lock member 121 and the second zipper-lock member 122 may stably surround the procedure tool.

Meanwhile, according to the first embodiment of the present disclosure, the guide zipper-lock unit 120 may further include a first pulley 127a and a second pulley 127b.

The first pulley 127a may be provided inside the case 123. The first zipper-lock member 121 may be wound around the first pulley 127a. When the operator holds and pulls the zipper-lock fixing portion 126 to which the leading end of the first zipper-lock member 121 in the length direction is fixed, the first zipper-lock member 121 may be unwound from the first pulley 127a.

The second pulley 127b may be provided inside the case 123. The second pulley 127b may be symmetric to the first pulley 127a. The second zipper-lock member 122 may be wound around the second pulley 127b. When the operator holds and pulls the zipper-lock fixing portion 126 to which the leading end of the second zipper-lock member 122 in the length direction is fixed, the second zipper-lock member 122 may be unwound from the second pulley 127b.

In this case, a coil spring (T) may be shaft-coupled to each of rotary shafts of the first pulley 127a and the second pulley 127b. Accordingly, automatic retraction of the first zipper-lock member 121 and the second zipper-lock member 122 that are slid out may be enabled.

That is, when the operator releases fastening of the zipper-lock fixing portion 126 fastened to the guide portion 125 of the guide zipper-lock unit 120 adjacent in the length direction, the first zipper-lock member 121 and the second zipper-lock member 122, which have been unwound by the coil springs (T), may be wound again around the first pulley 127a and the second pulley 127b, respectively.

At this time, the zipper-lock fixing portion 126, to which the leading ends of the first zipper-lock member 121 and the second zipper-lock member 122 in the length direction are fixed, may return to the initial position according to the winding of the first zipper-lock member 121 and the second zipper-lock member 122.

In addition, according to the first embodiment of the present disclosure, the guide zipper-lock unit 120 may further include coupling guiders 128.

The coupling guiders 128 may be provided in the case 123. In this case, the coupling guiders 128 may be positioned between the first pulley 127a and the second pulley 127b.

The coupling guiders 128 may guide the first zipper-lock member 121 unwound from the first pulley 127a and the second zipper-lock member 122 unwound from the second pulley 127b so as to meet and be engaged with each other in the length direction.

Accordingly, the first zipper-lock member 121 and the second zipper-lock member 122 may be slid out to the outside of the case 123 in an engaged state.

Before the first zipper-lock member 121 and the second zipper-lock member 122 are slid out to the outside, the leading ends in the length direction of the first zipper-lock member 121 and the second zipper-lock member 122, in which the cutouts 121a and 122a are formed, may be in close contact with each other in a central portion between the coupling guiders 128 so as to form a "U"-shaped groove.

Accordingly, a procedure tool connected to the other side of the connector 20, for example, the second procedure tool 12, may be vertically inserted in the "U"-shaped groove to be positioned between the first zipper-lock member 121 and the second zipper-lock member 122.

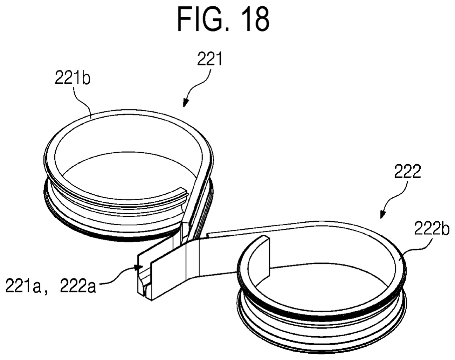

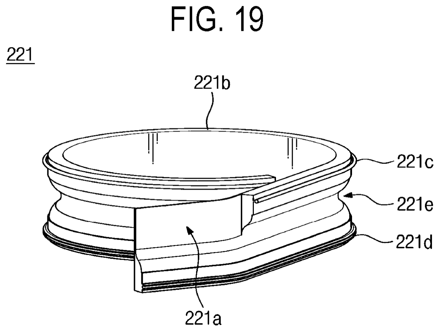

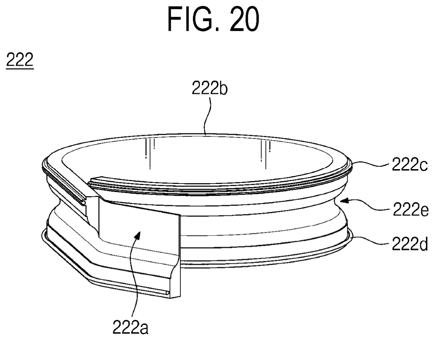

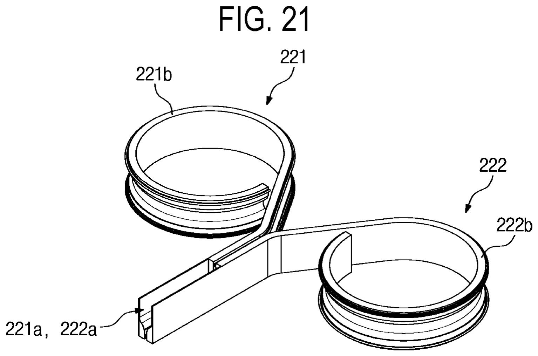

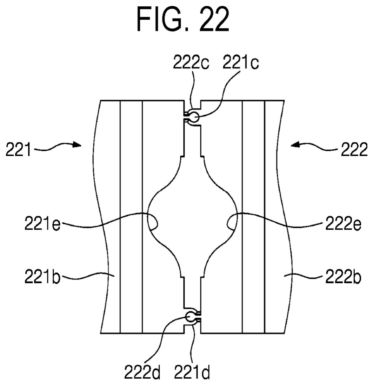

Hereinafter, a vascular intervention device according to a second embodiment of the present disclosure will be described with reference to FIGS. 18 to 22.

FIGS. 18 to 22 are views illustrating a first zipper-lock member and a second zipper-lock member of the vascular intervention device according to the second embodiment of the present disclosure.

In the second embodiment of the present disclosure, only the structures of the first zipper-lock member and the second zipper-lock member differ from those of the first embodiment of the present disclosure. Therefore, detailed descriptions of the remaining components will be omitted, and reference will be made to the reference numerals used in the previous drawings.

As illustrated in FIGS. 18 to 22, according to the second embodiment of the present disclosure, a guide zipper-lock unit 120 may include a first zipper-lock member 221 and a second zipper-lock member 222.

The first zipper-lock member 221 and the second zipper-lock member 222 may be provided so as to surround an outer circumferential surface of the procedure tool in the length direction while being progressively engaged with each other during sliding-out in the length direction of the procedure tool.

Accordingly, the procedure tool may be prevented from being exposed to the outside, thereby preventing in-body infection caused by contamination of the procedure tool.

According to the second embodiment of the present disclosure, the first zipper-lock member 221 may include a cutout 221a formed in a vertical direction at a leading end side in the length direction so that the first zipper-lock member 221 may be positioned between the procedure tool and the second zipper-lock member 222 while the procedure tool is inserted in the vertical direction. The cutout 221a may form a "U"-shaped groove that allows insertion of the procedure tool in the vertical direction when the cutout 221a meets the cutout 222a provided at a leading end side of the second zipper-lock member 222 in the length direction.

According to the second embodiment of the present disclosure, the first zipper-lock member 221 may include a first body 221b, a first protrusion 221c, a first socket portion 221d, and a first groove 221e.

The first body 221b forms an external appearance of the first zipper-lock member 221. The first body 221b may extend in one direction. In this case, the first body 221b may extend in a length capable of covering the procedure tool in the length direction. The first body 221b may be formed of a flexible material so as to be wound around a first pulley 127a.

The first protrusion 221c may be provided in the length direction on one side of one surface of the first body 221b, for example, on an upper side of the one surface. The first protrusion 221c may be fitted into a second socket portion 222c of the second zipper-lock member 222.

The first socket portion 221d may be provided in the length direction on the other side of the one surface of the first body 221b, for example, on a lower side of the one surface. A second protrusion 222d of the second zipper-lock member 222 may be fitted into the first socket portion 221d.

The first groove 221e may be provided in the length direction between the first protrusion 221c and the first socket portion 221d on the one surface of the first body 221b. The first groove 221e may form a seating hole in which the procedure tool is seated in the length direction when the first groove 221e meets a second groove 222e of the second zipper-lock member 222.

According to the second embodiment of the present disclosure, the second zipper-lock member 222 may be paired with the first zipper-lock member 221 to surround the procedure tool in the length direction, and thereby prevent the procedure tool from being exposed to the outside.

A cutout 222a may be provided at a leading end side of the second zipper-lock member 222 in the length direction so as to form a "U"-shaped groove that allows insertion of the procedure tool in the vertical direction when the cutout 222a meets the cutout 221a provided at the leading end side of the first zipper-lock member 221 in the length direction.

The second zipper-lock member 222 may include a second body 222b, a second socket portion 222c, a second protrusion 222d, and a second groove 222e.

The second body 222b forms an external appearance of the second zipper-lock member 222. The second body 222b may extend in one direction. In this case, the second body 222b may extend in a length that allows the procedure tool to be covered in the length direction. For example, the second body 222b may be provided to have the same length as the first body 221b of the first zipper-lock member 221.

The second body 222b may be formed of a flexible material so as to be wound around a second pulley 127b.

The second socket portion 222c may be provided in the length direction on one side of one surface of the second body 222b, for example, on an upper side of the one surface, which faces the one surface of the first body 221b. The second socket portion 222c may be provided so as to enable a fitting engagement of the first protrusion 221c, which is provided on the upper side of the one surface of the first body 221b.

The second protrusion 222d may be provided in the length direction on the other side of the one surface of the second body 222b, for example, on the lower side of the one surface.

The second protrusion 222d may be fitted into the first socket portion 221d provided on the lower side of the one surface of the first body 221b.

The second groove 222e may be provided in the length direction between the second socket portion 222c and the second protrusion 222d on the one surface of the second body 222b. The second groove 222e may form a seating hole in which the procedure tool is seated in the length direction when the second groove 222e meets the first groove 221e of the first zipper-lock member 221.

According to the second embodiment of the present disclosure, through a male-female engagement between the first protrusion 221c of the first zipper-lock member 221 and the second socket portion 222c of the second zipper-lock member 222, and a male-female engagement between the first socket portion 221d of the first zipper-lock member 221 and the second protrusion 222d of the second zipper-lock member 222, the first zipper-lock member 221 and the second zipper-lock member 222 may surround an outer circumferential surface of the procedure tool in the length direction while being progressively engaged with each other during sliding-out in the length direction of the procedure tool.

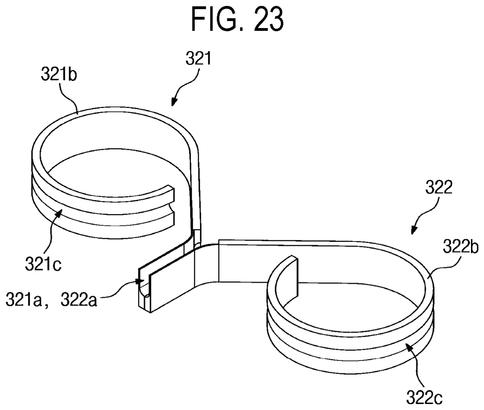

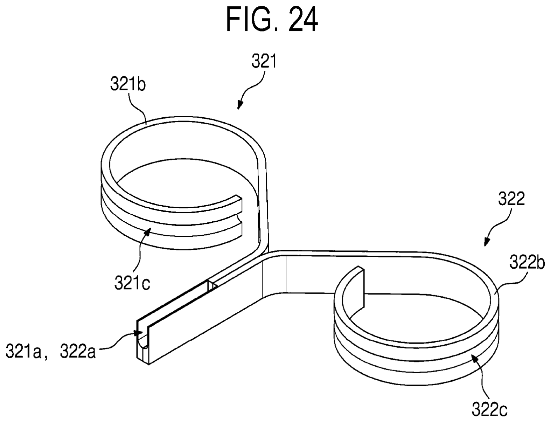

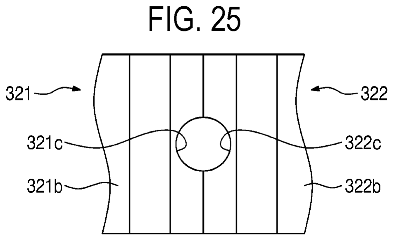

Hereinafter, a vascular intervention device according to a third embodiment of the present disclosure will be described with reference to FIGS. 23 to 25.

FIGS. 23 to 25 are views illustrating a first zipper-lock member and a second zipper-lock member of the vascular intervention device according to the third embodiment of the present disclosure.

In the third embodiment of the present disclosure, only the structures of the first zipper-lock member and the second zipper-lock member differ from those of the first embodiment of the present disclosure. Therefore, detailed descriptions of the remaining components will be omitted, and reference will be made to the reference numerals used in the previous drawings.

As illustrated in FIGS. 23 to 25, according to the third embodiment of the present disclosure, a guide zipper-lock unit 120 may include a first zipper-lock member 321 and a second zipper-lock member 322.

The first zipper-lock member 321 and the second zipper-lock member 322 may be provided so as to surround an outer circumferential surface of the procedure tool in the length direction while being progressively engaged with each other during sliding-out in the length direction of the procedure tool.

Accordingly, the procedure tool may be prevented from being exposed to the outside, thereby preventing in-body infection caused by contamination of the procedure tool.

According to the third embodiment of the present disclosure, the first zipper-lock member 321 may include a cutout 321a formed in a vertical direction at a leading end side in the length direction so that the first zipper-lock member 321 may be positioned between the procedure tool and the second zipper-lock member 322 while the procedure tool is inserted in the vertical direction. The cutout 321a may form a "U"-shaped groove that allows insertion of the procedure tool in the vertical direction when the cutout 321a meets the cutout 322a provided at a leading end side of the second zipper-lock member 322 in the length direction.

According to the third embodiment of the present disclosure, the first zipper-lock member 321 may include a first body 321b and a first groove 321c.

The first body 321b forms an external appearance of the first zipper-lock member 321. The first body 321b may extend in one direction. In this case, the first body 321b may extend in a length that allows the procedure tool to be covered in the length direction.

According to the third embodiment of the present disclosure, the first body 321b may be made of a rubber magnet.

The first groove 321c may be provided in the length direction on one surface of the first body 321b. The first groove 321c may form a seating hole in which the procedure tool is seated in the length direction when the first groove 321c meets a second groove 322c of the second zipper-lock member 322.

According to the third embodiment of the present disclosure, the second zipper-lock member 322 may be paired with the first zipper-lock member 321 to surround the procedure tool in the length direction, and thereby prevent the procedure tool from being exposed to the outside.

A cutout 322a may be provided at a leading end side of the second zipper-lock member 322 in the length direction so as to form a "U"-shaped groove that allows insertion of the procedure tool in the vertical direction when the cutout 322a meets the cutout 321a provided at the leading end side of the first zipper-lock member 321 in the length direction.

The second zipper-lock member 322 may include a second body 322b and a second groove 322c.

The second body 322b forms an external appearance of the second zipper-lock member 322. The second body 322b may extend in one direction. In this case, the second body 322b may extend in a length that allows the procedure tool to be covered in the length direction. For example, the second body 322b may be provided to have the same length as the first body 321b of the first zipper-lock member 321.

According to the third embodiment of the present disclosure, the second body 322b may be made of a rubber magnet. In this case, one surface of the first body 321b of the first zipper-lock member 321 made of a rubber magnet and one surface of the second body 322b of the second zipper-lock member 322, which face each other, may have opposite polarities.

The second groove 322c may be provided in the length direction on one surface of the second body 322b that faces the one surface of the first body 321b. The second groove 322c may form a seating hole in which the procedure tool is seated in the length direction when the second groove 322c meets the first groove 321c of the first zipper-lock member 321.

According to the third embodiment of the present disclosure, through magnetic coupling between the first body 321b of the first zipper-lock member 321 and the second body 322b of the second zipper-lock member 322, the first zipper-lock member 321 and the second zipper-lock member 322 may surround an outer circumferential surface of the procedure tool in the length direction while being progressively engaged with each other during sliding out in the length direction of the procedure tool.

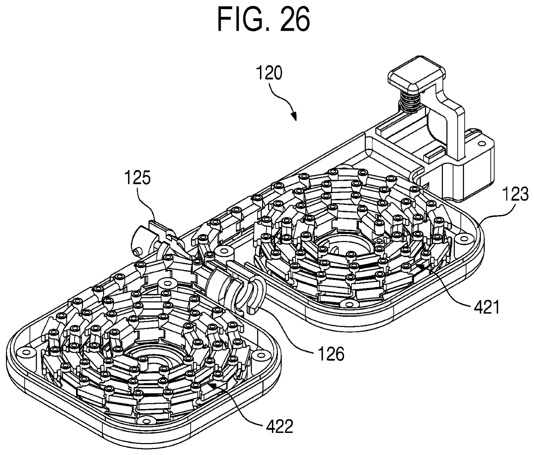

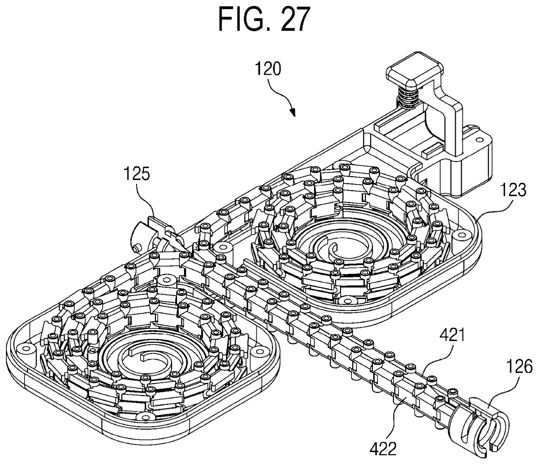

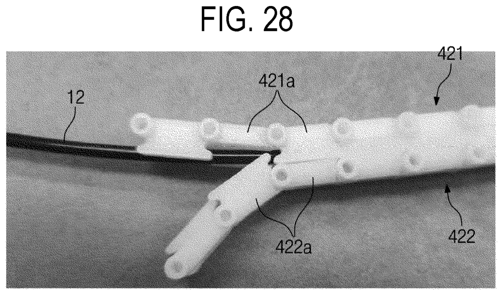

Hereinafter, a vascular intervention device according to a fourth embodiment of the present disclosure will be described with reference to FIGS. 26 to 28.

FIGS. 26 to 28 are views illustrating a first zipper-lock member and a second zipper-lock member of the vascular intervention device according to the fourth embodiment of the present disclosure.

In the fourth embodiment of the present disclosure, only the structures of the first zipper-lock member and the second zipper-lock member differ from those of the first embodiment of the present disclosure. Therefore, detailed descriptions of the remaining components will be omitted, and reference will be made to the reference numerals used in the previous drawings.

As illustrated in FIGS. 26 to 28, according to the fourth embodiment of the present disclosure, a guide zipper-lock unit 120 may include a first zipper-lock member 421 and a second zipper-lock member 422.

The first zipper-lock member 421 and the second zipper-lock member 422 may be configured to surround an outer circumferential surface of the procedure tool in the length direction while being progressively be engaged with each other during sliding-out in the length direction of the procedure tool.

Accordingly, the procedure tool may be prevented from being exposed to the outside, thereby preventing in-body infection caused by contamination of the procedure tool.

According to the fourth embodiment of the present disclosure, the first zipper-lock member 421 may include a plurality of first guide blocks 421a.

The plurality of first guide blocks 421a may extend in one direction while being joint-coupled to each other.

In this case, a first stepped portion (not illustrated) may be provided in the length direction on one surface of each first guide block 421a, such that, when the first guide blocks 421a are joint-coupled, the first stepped portions of the first guide blocks are connected in one direction.

According to the fourth embodiment of the present disclosure, the second zipper-lock member 422 may be paired with the first zipper-lock member 421 to surround the procedure tool in the length direction, and thereby prevent the procedure tool from being exposed to the outside.

The second zipper-lock member 422 may include a plurality of second guide blocks 422a.

The plurality of second guide blocks 422a may extend in one direction while being joint-coupled to each other.

The plurality of first guide blocks 421a forming the first zipper-lock member 421 and the plurality of second guide blocks 422a forming the second zipper-lock member 422 may have structures that are engaged with each other when they meet in the length direction.

Meanwhile, a second stepped portion (not illustrated) may be provided in the length direction on one surface of each second guide block 422a, such that, when the second guide blocks 421a are joint-coupled, the second stepped portions of the second guide blocks are connected in one direction. The second stepped portions (not illustrated) may form a seating hole in which the procedure tool is seated in the length direction when the second stepped portions meet the first stepped portions (not illustrated) of the first zipper-lock member 421.

According to the fourth embodiment of the present disclosure, through a multi-joint coupling formed when the plurality of first guide blocks 421a constituting the first zipper-lock member 421 and the plurality of second guide blocks 422a constituting the second zipper-lock member 422 meet in the length direction and are engaged with each other, the first zipper-lock member 421 and the second zipper-lock member 422 may surround an outer circumferential surface of the procedure tool in the length direction while being progressively be engaged with each other during sliding-out in the length direction of the procedure tool.

While the present disclosure has been described in detail using embodiments thereof, the scope of the present disclosure is not limited to the specific embodiments and should be interpreted based on the appended claims. A person ordinarily skilled in the art will appreciate that many modifications and variations can be made without departing from the scope of the present disclosure.

## Claims

1. A vascular intervention device comprising:
a main body extending in one direction; and
a plurality of guide zipper-lock units provided along a length direction of the main body and configured to guide a procedure tool inserted into a human body to an inside of the human body,
wherein the guide zipper-lock units each comprises a first zipper-lock member and a second zipper-lock member, and
wherein the first zipper-lock member and the second zipper-lock member are configured to surround an outer circumferential surface of the procedure tool in the length direction while being progressively engaged with each other during sliding-out in a length direction of the procedure tool.

2. The vascular intervention device of claim 1, wherein the main body comprises:
a rail frame extending in one direction;
a slide block slidably coupled to the rail frame in a length direction of the rail frame; and
a plurality of mounting portions provided in the length direction of the rail frame and the slide block, the mounting portions being configured such that the guide zipper-lock units are detachably mounted thereto,
wherein the mounting portions comprise fixed mounting portions respectively provided at leading ends of the rail frame and the slide block in the length direction, and a movable mounting portion provided on at least one side in the length direction of the slide block.

3. The vascular intervention device of claim 1, wherein the procedure tool is one of a catheter, a guidewire slid into an inner side of the catheter, a microcatheter slid into the inner side of the catheter, and a micro guidewire slid into an inner side of the microcatheter,
wherein each pair of the catheter and the guidewire, the catheter and the microcatheter, and the microcatheter and the micro guidewire is respectively connected to each other in the length direction through a connector, and
wherein the connector is mounted in the guide zipper-lock unit.

4. The vascular intervention device of claim 3, wherein each of the guide zipper-lock units further comprises:
a case configured to provide an internal mounting space, the case having an opening at an upper end thereof to allow a connector to be vertically mounted thereto, the connector having, in a length direction, one side to which one procedure tool is connected and another side to which another procedure tool is connected;
a guide portion provided on one side of the case and configured to guide the one procedure tool connected to the one side in the length direction of the connector, the guide portion having an upper end opened to allow the one procedure tool to be vertically inserted thereto; and
a zipper-lock fixing portion positioned on the another side of the case and configured to guide the another procedure tool connected to the another side in the length direction of the connector, the zipper-lock fixing portion having an upper side opened to allow the another procedure tool to be vertically inserted thereto, and being configured such that leading ends of the first zipper-lock member and the second zipper-lock member in the length direction are fixed thereto,
wherein the zipper-lock fixing portion is fastened to a guide portion of another guide zipper-lock unit adjacent in the length direction while being moved in the length direction together with the first zipper-lock member and the second zipper-lock member.

5. The vascular intervention device of claim 4, wherein the guide portion comprises fastening protrusions formed on opposite sides in a width direction,
wherein the zipper-lock fixing portion includes a first slit formed on one side in the width direction and connected to the opened upper end, and a second slit formed on another side in the width direction and connected to an opened lower end, and
wherein the fastening protrusions on the opposite sides of the guide portion adjacent in the length direction pass through the opened upper side and the opened lower side of the zipper-lock fixing portion, and one of the fastening protrusions is inserted into the first slit and another one of the fastening protrusion is inserted into the second slit.

6. The vascular intervention device of claim 4, wherein the guide zipper-lock units each further comprise:
a first pulley provided inside the case and configured such that the first zipper-lock member is wound therearound; and
a second pulley provided inside the case, arranged symmetrically with the first pulley, and configured such that the second zipper-lock member is wound therearound, and
wherein the first pulley and the second pulley have coil springs that are respectively shaft-coupled to rotation shafts of the first pulley and the second pulley so as to enable automatic retraction of the first zipper-lock member and the second zipper-lock member that are slid out.

7. The vascular intervention device of claim 6, wherein the guide zipper-lock units each further comprise a coupling guider, and
wherein the coupling guiders are provided between the first pulley and the second pulley and configured to guide the first zipper-lock member unwound from the first pulley and the second zipper-lock member unwound from the second pulley to meet and be engaged with each other in the length direction.

8. The vascular intervention device of claim 7, wherein the first zipper-lock member and the second zipper-lock member, which are fixed to the zipper-lock fixing portion and positioned between the coupling guiders, are provided, at leading end sides, with cutouts that are vertically formed so as to allow the another procedure tool to be inserted vertically and positioned between the first zipper-lock member and the second zipper-lock member.

9. The vascular intervention device of claim 1, wherein the first zipper-lock member comprises:
a first body extending in one direction and made of a flexible material;
a protrusion provided on one surface of the first body in a length direction; and
a first groove provided at a center of the protrusion in the length direction, and
wherein the second zipper-lock member comprises:
a second body extending in one direction and made of a flexible material;
a recess provided on one surface of the second body, which faces the one surface of the first body, in the length direction so as to allow the protrusion to be fitted thereto; and
a second groove provided in the length direction at a center of the recess and configured to form, together with the first groove, a seating hole in which the procedure tool is seated in the length direction when the second groove meets the first groove.

10. The vascular intervention device of claim 1, wherein the first zipper-lock member comprises:
a first body extending in one direction and made of a flexible material;
a first protrusion provided in the length direction on one side of one surface of the first body;
a first socket portion provided in the length direction on another side of the one surface of the first body; and
a first groove provided in the length direction between the first protrusion and the first socket portion on the one surface of the first body, and
wherein the second zipper-lock member comprises:
a second body extending in one direction and made of a flexible material;
a second socket portion provided in the length direction on one side of one surface of the second body, the one surface of the second body facing the one surface of the first body, and the second socket portion being configured to allow the first protrusion to be fitted thereto;
a second protrusion provided in the length direction on an opposite side of the one surface of the second body, the second protrusion being configured to be fitted into the first socket portion; and
a second groove provided in the length direction between the second socket portion and the second protrusion on the one surface of the second body, the second groove being configured to form, together with the first groove, a seating hole in which the procedure tool is seated in the length direction when the second groove meets the first groove.

11. The vascular intervention device of claim 1, wherein the first zipper-lock member comprises:
a first body extending in one direction and made of a rubber magnet; and
a first groove provided on one surface of the first body in the length direction, and
wherein the second zipper-lock member comprises:
a second body extending in one direction and made of a rubber magnet; and
a second groove provided in the length direction on one surface of the second body, which faces the one surface of the first body, the second groove being configured to form, together with the first groove, a seating hole in which the procedure tool is seated in the length direction when the second groove meets the first groove.

12. The vascular intervention device of claim 1, wherein the first zipper-lock member comprises a plurality of first guide blocks that extend in one direction while being joint-coupled to each other,
wherein the second zipper-lock member comprises a plurality of second guide blocks that extend in one direction while being joint-coupled to each other, and
wherein the plurality of first guide blocks and the plurality of second guide blocks have structures configured to be engaged with each other when they meet in the length direction.
